(19) **Europäisches Patentamt / European Patent Office / Office européen des brevets**

(11) **EP 1 458 390 B1**

(12) **EUROPEAN PATENT SPECIFICATION**

(45) Date of publication and mention
of the grant of the patent:
**13.08.2008 Bulletin 2008/33**

(51) Int Cl.:
*A61K 31/47* [(2006.01)]

(21) Application number: **02795443.7**

(22) Date of filing: **29.11.2002**

(86) International application number:
**PCT/KR2002/002247**

(87) International publication number:
**WO 2003/045389 (05.06.2003 Gazette 2003/23)**

(54) **A METHOD OF TREATING BACTERIAL INFECTIONS USING GEMIFLOXACIN OR A SALT THEREOF AND A CARBAPENEM ANTIBACTERIAL AGENT**

VERFAHREN ZUR BEHANDLUNG VON BAKTERIELLEN INFEKTIONEN MIT GEMIFLOXACIN ODER EINEM SEINER SALZE UND EINEM ANTIBAKTERIELLEN CARBAPENEM-MITTEL

PROCEDE DE TRAITEMENT D'INFECTIONS BACTERIENNES AU MOYEN DE GEMIFLOXACINE OU D'UN SEL DE CE COMPOSE ET D'UN AGENT ANTIBACTERIEN CARBAPENEME

(84) Designated Contracting States:
**AT BE BG CH CY CZ DE DK EE ES FI FR GB GR IE IT LI LU MC NL PT SE SK TR**

(30) Priority: **30.11.2001 US 335131 P**

(43) Date of publication of application:
**22.09.2004 Bulletin 2004/39**

(73) Proprietor: **LG Life Sciences Ltd.
Seoul 150-010 (KR)**

(72) Inventors:
• **NICONOVICH, Nancy**
**Collegeville, PA 19426 (US)**
• **RITTENHOUSE, Stephen**
**Collegeville, PA 19426 (US)**
• **McCLOSKEY, Lynn**
**Collegeville, PA 19426 (US)**
• **PAEK, Kyong-Sook**
**R & D Park, LG Life Sciences Ltd.
305-380 Taejeon (KR)**
• **KIM, Mu-Yong**
**R & D Park, LG Life Sciences, Ltd.
305-380 Taejeon (KR)**

(74) Representative: **Gillard, Richard Edward**
**Elkington and Fife LLP
Prospect House
8 Pembroke Road
Sevenoaks
Kent TN13 1XR (GB)**

(56) References cited:
• **WISE R. AND ANDREWS J.M.: 'The in-vitro activity and tentative breakpoint of gemifloxacin, a new fluoroquinolone' J. ANTIMICROB. CHEMOTHER. vol. 44, no. 5, November 1999, pages 679 - 688, XP002981605**
• **ZHANG L. ET AL: 'Fluoroquinolone susceptibilities of efflux-mediated multidrug-resistant pseudomonas aeruginosa, stenotrophomonas maltophilia and Burkholderia cepacia' J. ANTIMICROB. CHEMOTHER. vol. 48, no. 4, October 2001, pages 549 - 552, XP002981606**
• **HOBAN D.J. ET AL: 'A comparative in vitro surveillance study of gemifloxacin activities against 2,632 recent streptococcus pneumoniae isolates from across Europe, North America and South America. The gemifloxacin surveillance study research group' ANTIMICROB. AGENTS CHEMOTHER. vol. 44, no. 11, November 2000, pages 3008 - 3011, XP002981617**
• **HOBAN D.J. ET AL: 'Comparative in vitro activity of gemifloxacin, ciprofloxacin, levofloxacin and ofloxacin in a North American surveillance study' DIAGN. MICROBIOL. INFECT. DIS. vol. 40, no. 1-2, May 2001 - June 2001, pages 51 - 57, XP002981607**
• **FLUIT A.C. ET AL: 'Frequency of isolation and antimicrobial resistance of gram-negative and gram-positive bacteria from patients in intensive care units of 25 European university hospitals participating in the Eur. arm of the SENTRY antimicrobial surveillance program 97-98' EUR. J. CLIN. MICROBIOL. INFECT. DIS. vol. 20, no. 9, September 2001, pages 617 - 625, XP002981608**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 458 390 B1

- VISALLI M.A. ET AL: 'Determination of activities of levofloxacin, alone and combined with gentamicin, ceftazidine, cefpirome and meropenem, against 124 strains of pseudomonas aeruginosa by checkerboard and time-kill methodology' ANTIMICROB. AGENT CHEMOTHER. vol. 42, no. 4, April 1998, pages 953 - 955, XP002981609

- KUMAR A. ET AL: 'Ciprofoxacin, imipenem and rifampicin: in-vitro synergy of two and three drug combinations against pseudomonas cepacia' J. ANTIMICROB. CHEMOTHER. vol. 23, no. 6, June 1989, pages 831 - 835, XP002981618

**Description**

**TECHNICAL FIELD**

This invention relates to a novel method of treating bacterial infections using a combination of antibacterial agents, more particularly gemifloxacin or a salt thereof and a carbapenem antibacterial agent.

**BACKGROUND ART**

Gemifloxacin [(R,S)-7-(3-aminomethyl-4-methoxyiminopyrrolidin-1-yl)-1-cyclopropyl-6-fluoro-4-oxo-1,4-dihydro-1,8-naphthyridine-3-carboxylic acid] is a fluoroquinolone antibacterial agent which has enhanced in vitro antibacterial activity against Gram-positive bacteria, whilst retaining excellent activity against Gram negative bacteria. Without intending to be bound or limited by theory, gemifloxacin is believed to act via inhibition of bacterial topoisomerase II and IV. Gemifloxacin is highly selective for bacterial rather than human topoisomerase II.

EP 688772 discloses novel naphthyridine carboxylic acid derivatives, including gemifloxacin. WO 98/42705 discloses gemifloxacin mesylate and hydrates thereof including the sesquihydrate.

There still remains the need for improved methods of treating bacterial infections. In particular, infections caused by *Pseudomonas aeruginosa* continue to pose a therapeutic problem. In clinical practice, the combination of β-lactam antibiotic and aminoglycoside antibacterial agents has been shown to have an improved efficacy for the treatment of infections caused by *P. aeruginosa.* However, increased resistance of *P. aeruginosa* to aminoglycosides, coupled with their potential for nephrotoxicity, means there still remains the need for alternative treatments.

**DISCLOSURE OF THE INVENTION**

The present invention provides a composition for use in a method of treating bacterial infections which method comprises the separate, simultaneous or sequential administration to a patient in need thereof, of an effective amount of gemifloxacin or a salt thereof and a carbapenem antibacterial. The patient may be human or animal, and in a preferred embodiment is human.

The present inventors have found combinations of gemifloxacin or a salt thereof and a carbapenem antibacterial that provide an antibacterial regimen which has a broader spectrum of activity than either agent alone. In particular, the present invention includes such combinations having synergistic activity against several clinical isolates or reference strains of *Pseudomonas aeruginosa,* relative to either agent alone.

Suitable salts of gemifloxacin include those described in WO 98/42705, EP 688772, and US Patent No. 5,776,944. In particular embodiments, the salt of gemifloxacin is selected from the mesylate and hydrates thereof, in particular the sesquihydrate as described in WO 98/42705.

Suitable carbapenem antibacterials for use in the method of the invention are well known in the art and include, e.g., biapenem, imipenem, meropenem and panipenem.

In particular embodiments, the carbapenem antibacterial for use in the method of the invention is selected from meropenem and imipenem, in particular meropenem.

The carbapenem antibacterials referred to herein may be in the form of the free acids or pharmaceutically acceptable salts or in-vivo hydrolysable esters.

Gemifloxacin or a salt thereof and a carbapenem antibiotic, or a composition comprising same, may be used in accordance with the present invention to modulate metabolism of bacteria (e.g., clinical isolates, reference bacteria, pathogenic bacteria) and/or to treat infections caused by such bacteria. Clinical isolates or reference bacteria include *Streptococcus pneumoniae* (e.g., ATCC 49619), *Haemophilus influenzae* (e.g., ATCC 49247), *Moraxella catarrahalis* (e.g., 1502), *Staphylococcus aureus* (e.g., ATCC 29213), *Staphylococcus saprophyticus* (e.g., 662), *Klebsiella pneumoniae* (e.g. E70), *Proteus vulgaris* (e.g., ATCC 13315), *Enterococcus faecalis* (e.g., ATCC 29212), *Escherichia coli* (e.g., ATCC 25922), and *Pseudomonas aeruginosa* (e.g., ATCC 27853, 6016, 6156, 6168, P003, 6140, and PA018R).

Particular embodiments of the invention include the following treatment regimens:

> a) gemifloxacin and meropenem for the treatment of infections caused by *Pseudomonas aeruginosa;* and
> b) gemifloxacin and imipenem for the treatment of infections caused by *Pseudomonas aeruginosa.*

Suitably, gemifloxacin or a salt thereof and the carbapenem antibacterial are administered in a ratio of from about 10:1 to about 1:10, more suitably about 5:1 to 1:5, typically about 2:1w/w, expressed as the weight of the free acid and free base respectively.

Suitably, the administration is substantially simultaneous. This may conveniently be achieved by the co-administration of separate pharmaceutical compositions comprising gemifloxacin or a salt thereof and a carbapenem antibacterial. Such separate compositions may be usefully provided as a kit comprising a gemifloxacin, or a salt thereof, composition and a carbapenem antibacterial composition. The kit preferably contains sufficient dosages of gemifloxacin and the carbapenem antibacterial for a single course of therapy for the particular infection to be treated, together with instructions for administration.

Accordingly the present invention also provides a kit of parts for use in treating bacterial infections in mammals which comprises an antibacterially effective amount of (a) a pharmaceutical composition comprising gemifloxacin or a salt thereof, and a pharmaceutically acceptable carrier, and (b) a pharmaceutical composition comprising a carbapenem antibacterial and a pharmaceutically acceptable carrier.

Alternatively gemifloxacin, or a salt thereof, and a carbapenem antibacterial may be formulated together and administered in a single composition.

Accordingly in a further aspect the present invention further provides a pharmaceutical composition comprising gemifloxacin or a salt thereof, a carbapenem antibacterial, and a pharmaceutically acceptable carrier.

The present invention also includes the use of gemifloxacin or a salt thereof in the manufacture of a medicament for use in combination with a carbapenem antibacterial in the treatment of bacterial infections.

The invention further provides gemifloxacin or a salt thereof in combination with a carbapenem antibacterial for use in the treatment of bacterial infections.

The invention also includes a method of treating bacterial infections comprising administering to a mammal in need of such treatment, a therapeutically effective amount of gemifloxacin or a salt thereof and a carbapenem antibacterial. In preferred embodiments the treatment comprises administering therapeutically effective amounts of gemifloxacin or a salt thereof and a carbapenem antibiotic, wherein the activity of the antibiotics againt the bacterial infection is synergistic.

The invention also includes a method of modulating the metabolism of bacteria, comprising contacting the bacteria with an antibacterially effective amount of gemifloxacin and a salt thereof and a carbapenem antibiotic (optionally in the form of an antibacterially effective composition or kit of compositions, as described herein). Modulating metabolism suitably comprises inhibiting growth of the bacteria or killing the bacteria. Contacting the bacteria may suitably comprise the step of introducing the antibiotics or composition or kit of compositions comprising same into a mammal. In preferred embodiments, the method comprises contacting the bacteria with an antibacterially effective amount of gemifloxacin or a salt thereof and a carbapenem antibiotic, or composition or kit of compostions comprising same, wherein the activity of the antibiotics against the bacteria is synergistic.

Suitable formulations comprising gemifloxacin include those described in WO 98/42705, EP 688772, US Patent No. 5,776,944.

Suitable formulations comprising a carbapenem antibacterial are well known in the art and are readily available commercially.

Gemifloxacin or a salt thereof may be formulated with a carbapenem antibacterial and standard pharmaceutical carriers or diluents according to conventional procedures well known in the art. These procedures may involve mixing, granulating and compressing or dissolving the ingredients as appropriate to the desired preparation.

The invention further provides a method for the preparation of a pharmaceutical composition comprising gemifloxacin or a salt thereof and a carbapenem antibacterial which method comprises admixing the combination of gemifloxacin or a salt thereof and a carbapenem antibacterial and a pharmaceutically acceptable carrier.

The invention further provides a formulation for the treatment of antibacterial infections comprising gemifloxacin or a salt thereof and a carbapenem antibacterial. In preferred embodiments, the formulation comprises an amount of gemifloxacin or a salt thereof and carbapenem antibiotic wherein the activity of the antibiotics in treating the bacterial infection is synergistic.

The invention further provides the use of a formulation comprising gemifloxacin or a salt thereof and a carbapenem antibacterial in the manufacture of a medicament for the treatment of bacterial infections.

In the compositions, kits, and methods of the present invention the bacterial infection is preferably one caused by *P. aeruginosa.*

Infections caused by *P. aeruginosa* include wound infections, urinary tract infections and respiratory tract infections, together with general infections in an immunocompromised patient.

The contacting step and administration step in any of the methods of the invention may be performed in many ways that will be readily apparent to the skilled artisan. However, it is preferred that the contacting step and administration step is a provision of a composition comprising gemifloxacin or a salt thereof and a carbapenem antibiotic, or in the case of a kit according to the present invention, a composition comprising gemifloxacin or a salt thereof and a composition comprising a carbapenem antibiotic, to a human patient in need of such composition(s), or directly to bacteria in culture medium or buffer.

For example, when contacting a human patient or contacting said bacteria in a human patient *or in vitro,* the compositions or kit of compositions comprising gemifloxacin or a salt thereof and a carbapenem antibiotic, preferably pharmaceutical compositions, may be administered in any effective, convenient manner including, for instance, administration by topical, oral, anal, vaginal, intravenous, intraperitoneal, intramuscular, subcutaneous, intranasal or intradermal routes among others.

It is also preferred that these compositions be employed in combination with a non-sterile or sterile carrier or carriers for use with cells, tissues or organisms, such as a pharmaceutical carrier suitable for administration to a subject. Such compositions comprise, for instance, a therapeutically effective amount of gemifloxacin or a salt thereof, and/or a carbapenem antibiotic, a pharmaceutically acceptable carrier or excipient and optionally a media additive. Such carriers may include, but are not limited to, saline, buffered saline, dextrose, water, glycerol, ethanol and combinations thereof. The formulation should suit the mode of administration.

In therapy or as a prophylactic, the gemifloxacin or salt thereof and carbapenem antibiotic are preferably administered to an individual as an injectable composition (including compositions comprising both antibiotics, or separate injectable compositions comprising one or the other antibiotic), for example as a sterile aqueous dispersion, preferably an isotonic one.

Alternatively, the gemifloxacin or salt thereof and carbapenem antibiotic in the methods of the invention may be formulated for topical application for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

For administration to mammals, and particularly humans, it is expected that the antibacterially effective amount is a daily dosage level of the active agent from 0.001 mg/kg to 10 mg/kg, typically around 0.1 mg/kg to 1 mg/kg, preferably about 1 mg/kg. A physician, in any event, will determine an actual dosage that is most suitable for an individual and will vary with the age, weight and response of the particular individual. The above dosages are exemplary of the average case. There can, of course, be individual instances where higher or lower dosage ranges are merited, and such are within the scope of this invention. It is preferred that the dosage is selected to modulate metabolism of the bacteria in such a way as to inhibit or stop growth of said bacteria or by killing said bacteria. The skilled artisan may identify this amount as provided herein as well as using other methods known in the art, e.g. by the application MIC tests.

A further embodiment of the invention provides for the contacting step or administration step of the methods to further comprise contacting an in-dwelling device in a patient. In-dwelling devices include, but are not limited to, surgical implants, prosthetic devices and catheters, i.e., devices that are introduced to the body of an individual and remain in position for an extended time. Such devices include, for example, artificial joints, heart valves, pacemakers, vascular grafts, vascular catheters, cerebrospinal fluid shunts, urinary catheters, and continuous ambulatory peritoneal dialysis (CAPD) catheters.

The gemifloxacin or salt thereof and carbapenem antibiotic, or composition or kit of compositions of the invention, may be administered by injection to achieve a systemic effect against relevant bacteria, shortly before insertion of an in-dwelling device. Treatment may be continued after surgery during the in-body time of the device. In addition, the composition or kit of compositions could also be used to broaden perioperative cover for any surgical technique to prevent bacterial wound infections.

In addition to the therapy described above, gemifloxacin or a salt thereof and a carbapenem antibiotic, or composition or kit of compositions used in the methods of this invention may be used generally as a wound treatment agent to prevent adhesion of bacteria to matrix proteins, exposed in wound tissue and for prophylactic use in dental treatment as an alternative to, or in conjunction with, antibiotic prophylaxis.

Alternatively, gemifloxacin or a salt thereof and a carbapenem antibiotic, or composition or kit of compositions of the invention may be used to bathe an indwelling device immediately before insertion. The active agent will preferably be present at a concentration of $1\mu$g/ml to 10mg/ml for bathing of wounds or indwelling devices.

All documents cited or referred to herein, including issued patents, published and unpublished patent applications, and other publications are hereby incorporated herein by reference as though fully set forth.

**BEST MODE FOR CARRYING OUT THE INVENTION**

The invention will now be described by the following examples which are illustrative and not intended to limit the invention hereinbefore described.

**Examples**

The potential for the combination therapy of gemifloxacin and a carbapenem antibacterial was investigated.

1) <u>Gemifloxacin and Meropenem</u>

The combined effect of gemifloxacin and meropenem was investigated in P. *aeruginosa* by using a checkerboard titration method. Evaluation for synergistic activity was carried out by calculation of fractional inhibitory concentrations (FIC$_S$) according to the method of Eliopoulos et al (1996, Antimicrobial Combinations, Antibiotics in Laboratory Medicine, Victor Lorian, 4th Edition, pp 337-338, Baltimore, MD, Williams and Wilkins).

*P. aeruginosa* ATCC 27853 was obtained from the SmithKline Beecham AntiInfectives Research Culture Collection and stored at -80°C in 10% glycerol. Before testing, the isolate was passaged onto agar plates (trypticase soy agar containing 5% sheeps blood) from the frozen stock for two consecutive days. Cation adjusted Mueller Hinton broth (BBL, Cockeyville, MD) was used for the isolate.

Checkerboard microtitre broth dilution plates were prepared using the Hamilton MicroLab AT Plus system (Reno, NV). Two-fold serial dilutions (50 $\mu$l) of meropenem were made in columns 1 through 11 of a microtitre plate. Two-fold serial dilutions of gemifloxicin were prepared manually and the MicroLab AT Plus was used to dispense 25$\mu$l of gemifloxacin at each concentration into rows A through G of the microtitre plate. The concentration range tested varied for each drug/organism combination to encompass the MIC endpoints of the individual components. Column 12 contained two fold serial dilutions of gemifloxacin only, and row H contained two fold serial dilutions of meropenem only. The last well (H) in column 12 was used as a positive growth control well, containing only medium and test isolate.

The bacteria were diluted to a 0.5 McFarland standard and then further diluted 1/50. Each well was inoculated with 25 $\mu$l of the isolate to give a final inoculum density of approximately 5 x 10$^5$ cfu/ml. The MicroLab AT Plus 2 was used to add the inoculum to the microtitre plates. After inoculation, the plates were covered with a 96 well microtitre plate lid and incubated at 35°C in ambient air for 20-24 hours. A 10 $\mu$l aliquot of the inoculum was plated on trypticase soy agar containing 5% sheep blood to determine the purity of the final test inoculum.

Following incubation, a microtitre mirror reader (Cooke Instruments Ltd., England) was used to assist in determining the microdilution MIC endpoints. The MIC was determined as the lowest concentration of compound that inhibited visible growth of the organism.

The Fractional Inhibitory Concentrations (FICs) were calculated using the following formula:

$$(A)/(MIC\ A) + (B)/(MIC\ B) = FIC\ A + FIC\ B = FIC\ Index$$

wherein:

A is the MIC of drug A in the presence of drug B
B is the MIC of drug B in the presence of drug A
MIC A is the MIC of the organism to drug A alone
MIC B is the MIC of the organism to drug B alone
FIC A is the fractional inhibitory concentration of drug A
FIC B is the fractional inhibitory concentration of drug B

The FIC indices were interpreted using the following criteria:

$\leq 0.5$ = Synergy

> 0.5 - 1 = Additive

>1 - 2 = Indifference

>2 = Antagonism

Synergy was observed against *P. aeruginosa* 27853 for the gemifloxacin/meropenem combination.

| | | |
|---|---|---|
| gemifloxacin $\mu$g/ml | 0.125 | - |
| meropenem $\mu$g/ml | - | 0.125 |
| gemifloxacin MIC $\mu$g/ml | - | 0.125 |
| meropenem MIC $\mu$g/ml | 0.125 | - |

(continued)

| FIC | 0.5 | 0.5 |
|---|---|---|
| gemifloxacin MIC | 0.5 | 0.5 |
| meropenem MIC | 0.5 | 0.5 |

Other combinations of gemifloxacin/meropenem tested in a similar manner exhibited an additive or indifferent effect against *P. aeruginosa* 27853. In wells where no growth was observed and the MIC of one of the agents had been achieved or exceeded, the result was reported as inhibited.

2. Gemifloxacin and Imipenem

The combined effect of gemifloxacin and imipenem was investigated in P. *aeruginosa* by using a checkerboard titration method. The test organisms used in this study were all clinical isolates collected in Korea. The isolates were maintained frozen at -70°C prior to testing.

Gemifloxacin was tested at 12 concentrations (0.016 - 32 µg/ml) and imipenem was tested at 11 concentrations (0.016 - 16 µg/ml). Gemifloxacin was dispensed alone in the first row and combined with imipenem in the remaining rows. Imipenem solution was also dispensed alone in the first row in the first column. Test strains were grown for 18 h in Mueller-Hinton Broth, and then these overnight cultures were diluted with the same fresh medium to the density of approximately $10^7$ CFU/mL. The bacterial concentrations were determined by measuring optical density or turbidity of the suspension and were verified by standard colony counts on antibiotic-free agar plates. A diluted bacterial solution was applied to plates containing serially diluted antimicrobial agents to yield 5 X $10^5$ CFU/mL. Plates were incubated at 35°C for 18 hours.

Minimal inhibitory concentrations (MICs) for each separate drug were determined. For wells along the growth-no growth interface, synergy was determined by calculating the Fractional Inhibitory Concentration (FIC) index. The Fractional Inhibitory Concentration (FIC) indices were calculated using the following formula:

$$\text{FIC index} = \text{FIC}_A + \text{FIC}_B = [A]/\text{MIC}_A + [B]/\text{MIC}_B$$

Wherein:

   $\text{FIC}_{A(\text{or B})}$, is the FIC of drug A (or B)
   $\text{MIC}_{A(\text{or B})}$ is the MIC of the organism to drug A (or B)
   A (or B) is concentration of drug A (or B) that is the lowest inhibitory concentration

The FIC indices were interpreted using the following criteria:

   ≤ 0.5 = Synergy;
   > 0.5 - 1.0 = Additive;
   > 1.0 - 4.0 = Indifference;
   >4.0 = Antagonism

Synergy against P. aeruginosa 6168, 6140 and PA018R was observed for combinations of gemifloxacin and imipenem.

| P. aeruginosa | gemifloxacin MIC µg/ml | imipenem MIC µg/ml | $C_{\text{gemifloxacin}}$ MIC µg/ml | $C_{\text{imipenem}}$ MIC µg/ml | $\text{FIC}_A$ | $\text{FIC}_B$ |
|---|---|---|---|---|---|---|
| 6168 | 0.5 | 1 | 0.0625 | 0.25 | 0.13 | 0.25 |
| 6140 | 8 | 4 | 2 | 1 | 0.25 | 0.25 |
| PA018R | 4 | 16 | 1 | 1 | 0.25 | 0.06 |

Combinations of gemifloxacin and imipenem tested in a similar manner exhibited an additive effect against *P.*

*aeruginosa* 6016, 6156, and P003.

**INDUSTRIAL APPLICABILITY**

According to the present invention, combinations of gemifloxacin or a salt thereof and a carbapenem antibacterial provide an antibacterial regimen which has a broader spectrum of activity than either agent alone. In particular, such combinations has synergistic activity against several clinical isolates or reference strains of *Pseudomonas aeruginosa,* relative to either agent alone.

**Claims**

1.  Use of gemifloxacin or a salt thereof and a carbapenem antibacterial for the preparation of a medicament for treating bacterial infections by separate, simultaneous or sequential administration to a patient in need thereof, of an effective amount of gemifloxacin or a salt thereof and a carbapenem antibacterial.

2.  The use according to claim 1 comprising administration of gemifloxacin mesylate.

3.  The use according to claim 1 or 2 wherein the carbapenem antibacterial is meropenem or imipenem.

4.  The use according to claim 3 wherein the carbapenem antibacterial is meropenem.

5.  The use according to any one of the preceding claims wherein gemifloxacin or a salt thereof and a carbapenem antibacterial are administered in a ratio of from about 10:1 to about 1:10 (w/w).

6.  The use according to any one of the preceding claims wherein the bacterial infection is caused by *Pseudomonas aeruginosa.*

7.  The use according to any one of the preceeding claims in which an effective amount of gemifloxacin or a salt thereof and a carbapenem antibacterial are administered substantially simultaneously.

8.  The use according to claim 6 achieved by the the co-adminisiration of separate compositions comprising gemifloxacin or a salt thereof and a carbapenem antibacterial.

9.  A kit comprising a gemifloxacin, or a salt thereof, formulation and a carbapenem antibacterial formulation.

**Patentansprüche**

1.  Verwendung von Gemifloxacin oder einem Salz davon und einem antibakteriellen Carbapenem zur Herstellung eines Medikaments zur Behandlung bakterieller Infektionen durch separate, gleichzeitige oder aufeinander folgende Verabreichung einer wirksamen Menge an Gemifloxacin oder eines Salzes davon und eines antibakteriellen Carbapenems an einen dessen bedürfenden Patienten.

2.  Verwendung nach Anspruch 1, umfassend die Verabreichung von Gemifloxacinmesylat.

3.  Verwendung nach Anspruch 1 oder 2, worin das antibakterielle Carbapenem Meropenem oder Imipenem ist.

4.  Verwendung nach Anspruch 3, worin das antibakterielle Carbapenem Meropenem ist.

5.  Verwendung nach einem der vorhergehenden Ansprüche, worin Gemifloxacin oder ein Salz davon und das antibakterielle Carbapenem in einem Verhältnis von etwa 10:1 bis etwa 1:10 (w/w) verabreicht werden.

6.  Verwendung nach einem der vorhergehenden Ansprüche, worin die bakterielle Infektion durch *Pseudomonas aeruginosa* verursacht wird.

7.  Verwendung nach einem der vorhergehenden Ansprüche, worin eine wirksame Menge an Gemifloxacin oder einem Salz davon und dem antibakteriellen Carbapenem im Wesentlichen gleichzeitig verabreicht werden.

**8.** Verwendung nach Anspruch 6, die durch gleichzeitige Verabreichung separater, Gemifloxacin oder ein Salz davon und ein antibakterielles Carbapenem umfassender Zusammensetzungen erreicht wird.

**9.** Ein eine Gemifloxacin-Formulierung oder ein Salz davon und eine antibakterielle Carbapenem-Formulierung umfassender Kit.

**Revendications**

**1.** Utilisation de gémifloxacine ou d'un sel de celle-ci et d'un agent antibactérien à base de carbapénème pour la préparation d'un médicament permettant de traiter des infections bactériennes par administration séparée, simultanée ou séquentielle à un patient en ayant besoin, d'une quantité efficace de gémifloxacine ou d'un sel de celle-ci et d'un agent antibactérien à base de carbapénème.

**2.** Utilisation selon la revendication 1 comprenant l'administration de mésylate de gémifloxacine.

**3.** Utilisation selon la revendication 1 ou 2 dans laquelle l'agent antibactérien à base de carbapénème est le méropénème ou l'imipénème.

**4.** Utilisation selon la revendication 3 dans laquelle l'agent antibactérien à base de carbapénème est le méropénème.

**5.** Utilisation selon l'une quelconque des revendications précédentes dans laquelle la gémifloxacine ou un sel de celle-ci et un agent antibactérien à base de carbapénéme sont administrés selon un rapport allant d'environ 10/1 à environ 1/10 (p/p).

**6.** Utilisation selon l'une quelconque des revendications précédentes dans laquelle l'infection bactérienne est provoquée par *Pseudomonas aeruginosa.*

**7.** Utilisation selon l'une quelconque des revendications précédentes dans laquelle une quantité efficace de gémifloxacine ou d'un sel de celle-ci et d'un agent antibactérien à base de carbapénème est administrée de façon sensiblement simultanée.

**8.** Utilisation selon la revendication 6 obtenue par l'administration conjointe de compositions distinctes comprenant de la gémifloxacine ou un sel de celle-ci et un agent antibactérien à base de carbapénéme.

**9.** Kit comprenant une formulation de gémifloxacine ou d'un sel de celle-ci et une formulation d'un agent antibactérien à base de carbapénème.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 688772 A
- WO 9842705 A
- US 5776944 A

**Non-patent literature cited in the description**

- **ELIOPOULOS et al.** Antimicrobial Combinations, Antibiotics in Laboratory Medicine. Williams and Wilkins, 1996, 337-338